# EUROPÄISCHE PATENTSCHRIFT

(11) **EP 0 454 621 B1**
(45) Veröffentlichungstag und Bekanntmachung des Hinweises auf die Patenterteilung: **12.07.2000**
(21) Anmeldenummer: 91810284.9
(22) Anmeldetag: 16.04.1991
(51) Int. Cl.: C07D 275/04, A01N 43/80, C07C 323/22

(54) **Nematizide Mittel**
Nematicide agents
Composés nématocides

(30) Priorität: 23.04.1990 CH 135890; 28.01.1991 CH 25691
(43) Veröffentlichungstag der Anmeldung: 30.10.1991
(73) Patentinhaber: Novartis AG, 4058 Basel (CH); Novartis-Erfindungen Verwaltungsgesellschaft m.b.H., 1235 Wien (AT)
(72) Erfinder: Sutter, Marius, Dr., CH-4058 Basel (CH); Kunz, Walter, Dr., CH-4104 Oberwil (CH)

(56) Entgegenhaltungen:
- DE-A- 1 915 387
- FR-A- 2 002 891

## Beschreibung

Die vorliegende Erfindung betrifft ein Verfahren zur Bekämpfung von Nematoden, insbesondere von pflanzenschädigenden Nematoden.

Die Erfindung betrifft ferner neue, nematizid wirksame Benzisothiazole und Verfahren zu ihrer Herstellung.

Die nematizid wirksamen Benzisothiazole gemäss der vorliegenden Erfindung entsprechen der allgemeinen Formel I 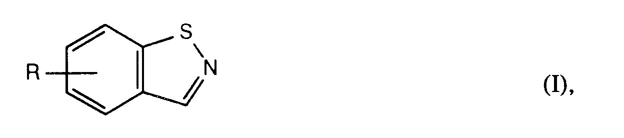 worin
R Nitro oder Halogen bedeutet.

Als Halogen kann hier und in der folgenden Beschreibung Fluor, Chlor, Brom oder Jod figurieren.

Die als Nematizide bekannten Verbindungen haben bisher die in der Praxis an sie gestellten Ansprüche nicht im vollen Umfang befriedigen können.

Ziel der vorliegenden Erfindung war es daher ein neues Verfahren zur Bekämpfung von Nematoden sowie, neue nematizid Mittel mit vorteilhaften Eigenschaften bereitzustellen.

Durch die Bereitstellung der erfindungsgemässen Mittel und Verfahren, welche als Wirkstoff die Verbindungen der Formel I und zusätzlich mindestens noch einen Träger enthalten, ist es nun gelungen, einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle, Mais, Reis und Gemüse sowie Schäden in Baumschulen und im Zierpflanzenbau nachhaltig eingedämmt werden. Die erfindungsgemässen Mittel zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Mitteln die Nematodengattungen Ditylenchus (Stengelparasiten) Aphelenchoides (Blattnematoden) und Anguina (Blütennematoden) wirkungsvoll bekämpfen.

Mit den Mitteln, welche als Wirkstoff die Verbindungen der Formel I enthalten, lassen sich vorzugsweise besonders schädliche Nematodenarten der Gattung Meliodogyne, wie z.B. Meliodogyne incognita, sowie der Gattung Heterodera, wie z.B. Heterodera glycines (Sojabohnen-Zystennematode) erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Mittel wird durch eine relativ geringe Phytotoxizität in vorteilhafter Weise begleitet. Im Vergleich mit bekannten nematiziden Mitteln weisen die erfindungsgemässen Mittel eine nur geringe Human-Toxizität auf.

Eine nematizide Wirksamkeit wird durch Hemmung der Wurzelgallbildung an der behandelten Kulturpflanze im Vergleich zur unbehandelten Pflanze festgestellt und auch bestimmt.

Die Wirkung wird als "gut" bezeichnet, wenn der Befall der behandelten Pflanze geringer ist als 20 % des Befalls der unbehandelten Pflanze.

Von den Verbindungen der Formel I werden als Nematizide jene der Formel I', 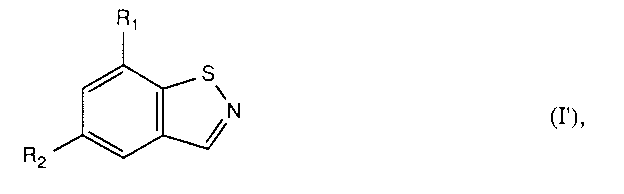 bevorzugt, worin
R₁ Wasserstoff oder Halogen bedeutet,
R₂, wenn R₁ für Wasserstoff steht, Nitro, wenn R₁ für Halogen steht, Wasserstoff bedeutet.

Von den Verbindungen der Formel I weisen jene der Formel I˝ 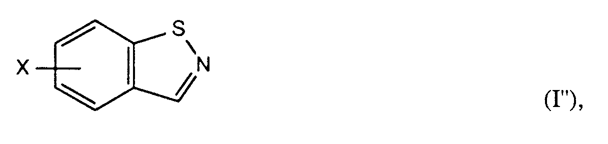 worin
X Brom, Fluor oder Jod bedeutet, eine bemerkenswerte nematizide Aktivität auf.

Diese Verbindungen sind neu und sind Gegenstand der vorliegenden Erfindung. Von den letztgenannten Verbindungen sind die der Formeln Ia, Ic, Id und Ie 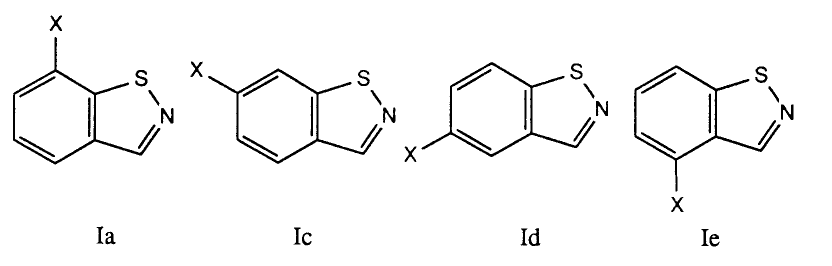 hervorzuheben.

Von den Verbindungen der Formel I werden wegen iher nematiziden Aktivität das 5-Nitrobenzisothiazol und das 7-Chlorbenzisothiazol ebenfalls bevorzugt.

Das 5-Nitrobenzisothiazol und ein Verfahren zu seiner Herstellung ist in der DE-OS 3,018,108 beschrieben. In dieser Publikation wird offenbart, dass die Verbindungen der Formel 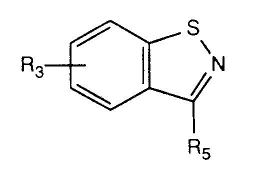 worin R₃ Wasserstoff, einen aliphatischen, cycloaliphatischen oder einen, gegebenenfalls anellierten, aromatischen Rest, Halogen, eine Alkoxygruppe, Nitrogruppe oder den Rest 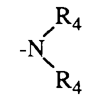 bezeichnet, worin R₄ jeweils Wasserstoff, einen aliphatischen, cycloaliphatischen, araliphatischen oder aromatischen Rest bedeuten, und R₅ für Wasserstoff, einen aromatischen oder heterocyclischen Rest steht, wertvolle Zwischenprodukte für die Herstellung von Farbstoffen, Pharmazeutika u.a. sind.

Das 7-Nitrobenzisothiazol ist aus der Litertur [J. Chem. Soc. Perkin Trans. I, 385 (1984)] bekannt. Es entsteht auch neben 4- und 5-Nitrobenzisothiazol bei der Nitrierung von Benzisothiazol mit Kaliumnitrat in konzentrierter Schwefelsäure.

In der Patentschrift CH 539385 wird erwähnt, dass in 3-Stellung substituierte Benzisothiazole herbizide Wirkung besitzen.

In der Publikation FR 2,002,891 werden für die Bekämpfung von Insekten und Akarina geeignete Benzisothiazole der Formel 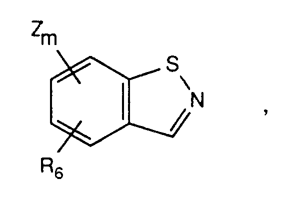 worin Z Nitro oder Halogen, m die Zahlen 1 oder 2 und R₆ Wasserstoff oder Alkyl bedeuten, beschrieben. Nitrobenzisothiazole werden nicht aufgeführt.

Vier Monochlorbenzisothiazole sind bekannt; das 4-Chlorbenzisothiazol aus Liebigs Ann. Chim 768 (1980) und ibid 729, 146 (1969), das 5-Chlorbenzisothiazol aus der DE-OS 3,018,108 und das 6-Chlorbenzisothiazol aus Ann. Chim. (Rome) 53, 577 (1963); das 7-Chlorbenzisothiazol ist in der Ann. Chim. (Rome) 53 (12) 1860-8 (1963), CA 60, 12000d beschrieben. Die Verbindung wurde ausgehend von 7-Aminobenzisothiazol über das Diazoniumchlorid durch Sandmeyer-Reaktion, in Gegenwart von Cu(I)Cl hergestellt.

Während nematizid wirksame Thiadiazole aus der EP-O 321408 und EP-O 217417 bekannt sind, werden Isothiazole oder Benzisothiazole mit dieser Eigenschaft in der Literatur nicht erwähnt.

Die neuen Verbindungen der Formel I'' können hergestellt werden, indem man ein Aminobenzisothiazol der Formel II zum Diazoniumsalz der Formel III diazotiert und letzeres, gegebenenfalls in Gegenwart von Cu(I)X oder Fe(II)X₂ mit der entsprechenden Halogenwasserstoffsäure der Formel HX umsetzt, 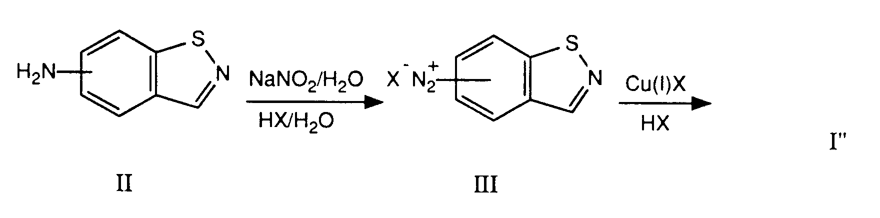 wobei X Brom, Fluor oder Jod bedeutet.

Hervorzuheben ist die Herstellung der neuen Verbindungen der Formel Ia aus 7-Aminobenzisothiazol nach diesem Verfahren.

Im Falle der Fluorsubstitution (X = F) verläuft die Umsetzung in wasserfreier Flussäure glatt. Eine andere Möglichkeit zur Herstellung der Fluorverbindungen der Formel Ia (X = F) besteht in der thermischen Zersetzung des leicht isolierbaren Diazonium-tetra-fluoroborates der Formel III (X⁻= BF₄⁻). Diese Arbeitsweise ist am Beispiel von analogen Reaktionsfolgen in Houben-Weyl's "Die Methoden der Organischen Chemie", Bd. 5/3, S. 213 ff. (1962) beschrieben.

Im Falle der Jodsubstitution (X = J) kann das Cu(I)J durch KJ ersetzt werden.

Die Diazotierung des aromatischen Amins der Formel II erfolgt nach bekannten Methoden, vorzugsweise mit Alkalinitrit und Halogenwasserstoffsäure in kalter wässriger Lösung. Die Überführung des Diazoniumsalzes in die Halogenverbindung der Formel Ia erfolgt nach den bekannten Prinzipien der Sandmeyer-Reaktion, siehe Houben-Weyl, "Die Methoden der Organischen Chemie", Bd. 5/4, S. 437 ff., 639 ff. (1960); ibidem, Bd 5/3, S. 213., (1962).

Die neuen Verbindungen der Formel I'' 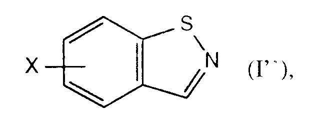 worin X Brom, Fluor oder Jod bedeutet, können nach einem neuen Verfahren hergestellt werden, indem man ein substituiertes Halo-benzaldehyd der Formel V mit einem Thioalkohol R₉SH in Lösung, in Gegenwart einer Base zum Thioether der Formel VI umsetzt und letzteren, mit oder ohne Isolierung, mit Hydroxylamin-O-sulfonsäure in Lösung umsetzt und zum erwünschten Produkt zyklisiert, 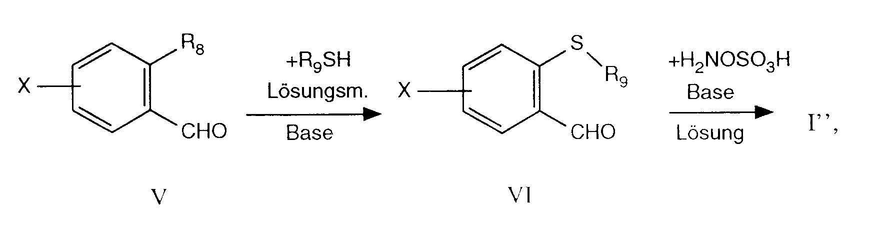 wobei X Brom, Fluor oder Jod, R₈ Halogen und R₉ C₁-C₃₀-Alkyl, C₃-C₇-Cycloalkyl oder C₇-C₉-Aralkyl bedeuten.

Gemäss einer bevorzugten Ausführungsform des Verfahrens wird der Thioether der Formel VI mit Hydroxylamin-O-sulfonsäure in Lösung umgesetzt und durch Zugabe einer Base zum erwünschten Produkt zyklisiert.

Unter Alkyl werden hier und in der folgenden Beschreibung gerade und verzweigtkettige Gruppen, wie beispielsweise Aethyl, Propyl, Butyl, Isobutyl oder tert.-Butyl verstanden.

Von den Mercaptanen werden C₁-C₄-Alkylmercaptane oder das Benzylmercaptan bevorzugt.

Die Reaktion kann vorteilhaft auch in Gegenwart von Metallsalzen wie z.B. Kupfer, Nickel oder Palladiumsalze durchgeführt werden.

Als Base für die erste Stufe dieser Reaktionsfolge eignen sich beispielsweise Hydride, Amide, Alkoholate, Carbonate oder Hydrogencarbonate von Alkali- oder Erdalkalimetallen, vorzugsweise Kalium- oder Natriumcarbonat. Vorteilhaft ist die Reaktion in Alkoholen oder in aprotischen Lösungsmitteln, wie z.B. Dimethylformamid, Dimethylsulfoxid, Dimethylpropylenharnstoff oder Dimethylethylenharnstoff oder in Äthern, wie Diethyläther oder Tetrahydrofuran bei 0-70°C, vorzugsweise bei Raumtemperatur durchzuführen.

Als Base für die zweite Stufe eignen sich Alkalicarbonate- oder -hydrogencarbonate, beispielsweise Natriumhydrogencarbonat. Als für die zweite Stufe geeignete Basen können noch tert.Amine, beispielsweise Triäthylamin oder Pyridin genannt werden.

Dieses Verfahren ist neu und ist Gegenstand der Erfindung. Auch die Zwischenprodukte der Formel VI, worin X Brom, Fluor oder Jod und R₉ C₁-C₃₀-Alkyl, C₃-C₇-Cycloalkyl oder C₇-C₉-Aralkyl bedeuten sind neu und bilden Gegenstand der vorliegenden Erfindung.

Von den Verbindungen der Formel VI sind als Zwischenprodukte jene hervorzuheben, bei welchen R₉ C₁-C₆-Alkyl oder den Benzylrest bedeutet.

Die Ausgangsprodukte der erwähnten Herstellungsverfahren sind entweder bekannte, im Handel erhältliche Verbindungen oder können nach bekannten Verfahren hergestellt werden.

Für die zweite Stufe eignen sich aprotische organische Lösungsmittel, wie beispielsweise Methylenchlorid, Ether, wie beispielsweise Diethylether, Dioxan oder Tetrahydrofuran, Ester, wie beispielsweise Ethylacetat, Kohlenwasserstoffe, wie beispielsweise Hexan, Cyclohexan, Methylcyclohexan oder Toluol und deren Mischungen mit Wasser. Die Reaktionstemperatur der zweiten Stufe liegt zwischen -20 und 100°C, vorzugsweise bei 20 bis 50°C.

Das erfindungsgemässe neue Verfahren eignet sich insbesondere zur Herstellung der Verbindungen der Formel Ia 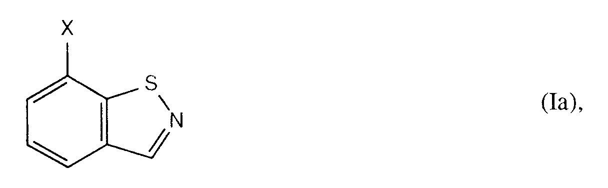 wobei man ein 2,3-Dihalo-benzaldehyd der Formel VII mit Benzylmercaptan in Lösung, in Gegenwart einer Base zum Thioether der Formel VIII umsetzt und letzteren, mit oder ohne Isolierung, mit Hydroxylamin-O-sulfonsäure in Lösung umsetzt und zum erwünschten Produkt zyklisiert, 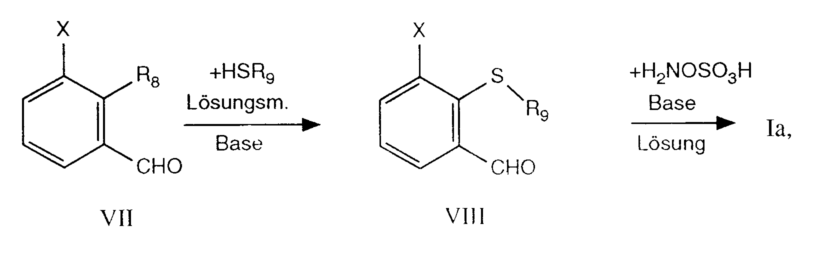 worin X Brom, Fluor oder Jod, R₈ Hingen und R₉ den Benzylrest bedeuten.

Gemäss einer bevorzugten Ausführungsform des Verfahrens wird der Thioether der Formel VIII mit Hydroxylamin-O-sulfonsäure in Lösung umgesetzt und durch Zugabe einer Base zum erwünschten Produkt zyklisiert.

Gemäss einer besonders bevorzugten Ausführungsform wird das Zwischenprodukt der Formel VIII mit Hydroxylamin-O-sulfonsäure bei pH = 5 umgesetzt und die Zyklisierung durch anschliessende Basenzugabe durchgeführt.

Das Verfahren ist bevorzugt, wenn R₈ Chlor bedeutet.

Darüber hinaus schliesst die vorliegende Erfindung zusätzlich die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen. Die erfindungsgemässen Mittel enthalten dementsprechend eine wirksame Menge mindestens einer der Verbindungen der Formel I.

Auch ein Verfahren zur Behandlung von Pflanzen, das durch die Applikation der Verbindungen der Formel I oder der neuen Mittel charakterisiert ist, ist in die Erfindung eingeschlossen.

Ein bevorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugs-weise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 0,1 bis 10 kg Aktivsubstanz (AS) je ha; bevorzugt 0,3 bis 5 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsminel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuss-oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol- Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen, in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien.

Die agrochemischen Zubereitungen enthalten eine wirksame Menge, d.h. in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

### H. Herstellungsbeispiele:

H.1 Eine aus 500 ml Dimethylformamid, 100 g 2,3-Dichlorbenzaldehyd und 100 g Kaliumcarbonat bestehende Suspension wird mit 65 ml Benzylmercaptan versetzt und bei 20-40°C über Nacht gerührt. Anschliessend wird das Reaktionsgut mit Wasser und Diethylether verdünnt und verrührt. Nach der Schichtentrennung wird die etherische Lösung mit gesättigter Kochsalzlösung gewaschen, der Ether wird verdampft und der Rückstand wird aus Ethanol umkristallisiert. Der erhaltene 2-Benzylthio-3-chlorbenzaldehyd schmilzt bei 73-74°C, die Ausbeute beträgt 118 g (90 % d. Th.).

101 g 2-Benzylthio-3-chlorbenzaldehyd, 47 g Hydroxylamin-O-sulfonsäure und 6 g Natriumsulfat werden in 200 ml Wasser während einer Stunde gerührt. Nach Zugabe von weiteren 100 ml Wasser und 100 ml Methylenchlorid werden 120 g Natriumhydrogencarbonat allmählich, bei gleichmässiger Gasentwicklung, zugegeben. Nach weiteren 1½ Stunden wird mit Methylenchlorid extrahiert, die organische Schicht mit Sole gewaschen und das Lösungsmittel im Vakuum verdampft.

Der Rückstand wird in Hexan/Essigester = 95:5 gelöst und die Lösung wird über Kieselgel chromatographiert.

Nach Verdampfen des Lösungsmittels erhält man 62,8 g 7-Chlorbenzisothiazol, Verb. Nr. 2, (Tabelle). Schmelzpunkt: 50-51°C.

### H.2 4-Nitrobenzisothiazol, 5-Nitrobenzisothiazol und 7-Nitrobenzisothiazol

Zu 750 ml konzentrierter Schwefelsäure wird unter Kühlung 300 g Benzisothiazol zugegeben, die Lösung wird auf -40°C abgekühlt und portionenweise mit 227,5 g Kaliumnitrat versetzt, so dass die Temperatur nicht über Raumtemperatur steigt. Nach 15 Stunden Stehen bei Raumtemperatur wird das Reaktionsgut auf Eis gegossen, der Festanteil abfiltriert und getrocknet. Das Produkt wird an Kieselgel mit Tetrahydrofuran:Methylenchlorid:Hexan 5:10:85 chromatographiert. Aus den Fraktionen werden nach Verdampfen 5,5 % d.Th. 4-Nitrobenzisothiazol 56,5 % d.Th. 5-Nitrobenzisothiazol und 36,8 % d.Th. 7-Nitrobenzisothiazol isoliert, (Verbindungen 16, 11 und 1, Tabelle).

### H.3 7-Brombenzisothiazol

Zu einer Suspension von 10 g 7-Aminobenzisothiazol in 30 ml Wasser wird 100 ml 3N Bromwasserstoffsäure zugegeben. Zu der resultierenden auf 5°C gekühlten Suspension wird eine Lösung von 4,6 g Natriumnitrit in 10 ml Wasser zugetropft und während 3h bei 0°C gerührt. Die entstandene Lösung wird zu einer 50°C warmen Lösung von 12,4 g Kupfer(I)bromid in 230 ml 3N Bromwasserstoffsäure zugetropft. Das nach Wasserdampfdestillation erhältliche Produkt wird durch Chromatographie an Kiselgel mit Toluol:Essigester 4:1 als Laufmittel gereinigt. Die Ausbeute beträgt 35 % d.Th. 7-Brombenzisothiazol, Verbindung Nr. 3, (Tabelle).

### H.4 7-Fluorobenzisothiazol

Zu einer Suspension von 2,5 g 7-Aminobenzisothiazol in 45 ml Wasser und 23 ml konzentrierter Salzsäure wird bei 5°C eine Lösung von 1,14 g Natriumnitrit in 30 ml Wasser zugetropft. Nach 30 Minuten Rühren bei 5°C wird die Suspension filtriert und das Filtrat sofort mit 23 ml einer 40%igen Lösung von Natriumtetrafluoroborat in Wasser versetzt. Nach 1h bei 0°C werden die gebildeten Kristalle abfiltriert und an der Luft getrocknet. Thermische Zersetzung dieser Kristalle bei 140 bis 160°C gefolgt von einer Destillation ergibt 1,2 g, 7-Fluorobenzisothiazol Verbindung Nr. 4, (Tabelle).

### H.5 7-Jodbenzisothiazol

Zu einer Suspension von 3,0 g 7-Aminobenzisothiazol in 30 ml Wasser und 67 ml 1N Salzsäure wird bei 5°C eine Lösung von 1,4 g Natriumnitrit in 50 ml Wasser zugetropft und 1 h bei 0°C gerührt. Diese Lösung wird zu einer Mischung aus 10,6 g Kaliumiodid, 150 ml Wasser und 150 ml Chloroform zugetropft. Nach 30 Minuten wird das Reaktionsgemisch basisch gestellt, die organische von der wässrigen Phase abgetrennt und getrocknet. Nach Reinigung durch Chromatographie an Kieselgel mit Toluol:Essigester 4:1 werden 1,1 g 7-Iodbenzisothiazol, Verbindung Nr. 5, (Tabelle), isoliert.

Nach den angegebenen Methoden können folgende Verbindungen hergestellt werden:

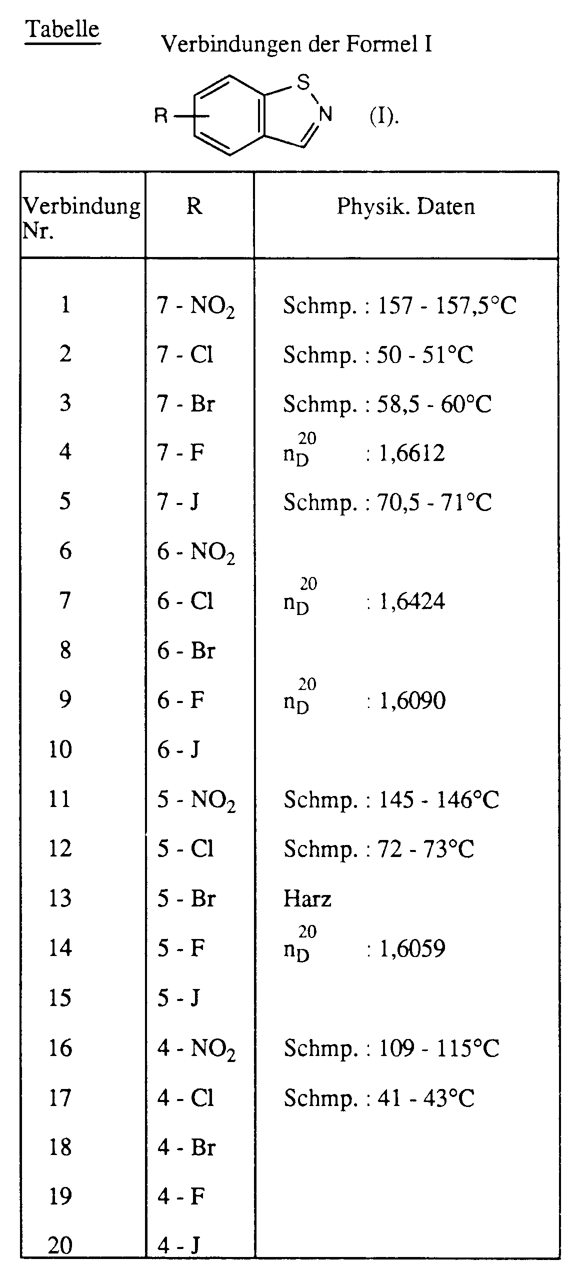

### F. Formulierungsbeispiele für flüssige Wirkstoffe der Formel I (% = Gewichtsprozent)

| F. 1 Emulsions-Konzentrate | a) | b) | c) |
|---|---|---|---|
| Wirkstoff aus der Tabelle | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykol-ether (30 Mol Ethylenoxid) | - | - | 12 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F.2 Lösungen | a) | b) | c) | d) |
|---|---|---|---|---|
| Wirkstoff aus der Tabelle | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |
| (MG = Molekulargewicht) | | | | |

Die Lösungen sind zur Anwendung in Form kleinster Tropfen geeignet.

| F.3 Granulate | a) | b) |
|---|---|---|
| Wirkstoff aus der Tabelle | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F.4 Stäubemittel | a) | b) |
|---|---|---|
| Wirkstoff aus der Tabelle | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

### Formulierungsbeispiele für feste Wirkstoffe der Formel I (% = Gewichtsprozent)

| F.5 Spritzpulver | a) | b) | c) |
|---|---|---|---|
| Wirkstoff aus der Tabelle | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F.6 Emulsions-Konzentrat | |
|---|---|
| Wirkstoff aus der Tabelle | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F.7 Stäubemittel | a) | b) |
|---|---|---|
| Wirkstoff aus der Tabelle | 5 % | 8 % |
| Taltum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| F.8 Extruder Granulat | |
|---|---|
| Wirkstoff aus der Tabelle | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| F.9 Umhüllungs-Granulat | |
|---|---|
| Wirkstoff aus der Tabelle | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| F. 10 Suspensions-Konzentrat | |
|---|---|
| Wirkstoff aus der Tabelle | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen | 0,8 % |
| wässrigen Emulsion Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### B. Biologische Beispiele

### B. 1 Wirkung gegen Meloidogyne incognita auf Tomaten

Eier von Meloidogyne incognita werden in Sand eingemischt Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26 ± 1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Die Verbindungen aus der Tabelle zeigen gegen Meloidogyne incognita durch Reduktion der Wurzelgallbildung eine Wirksamkeit. Unbehandelte jedoch infizierte Kontrollpflanzen weisen dagegen starke Wurzelgallbildung auf (= 100 %). Eine gute Wirkung zeigen die Verbindungen der Formel I mit weniger als 20 % Restbefall, die Verbindungen Nr. 2 und 11 z.B. hemmen im obigen Versuch die Wurzelgallbildung sogar fast vollständig (0-10 % Restbefall).

### B.2 Wirkung gegen Heterodera glycines auf Sojabohnen

Sandige Erde wird mit Eiern des Sojabohnenzystennematoden *H. glycines* infestiert, etwa 6000 Eier pro Topf. Anschliessend werden die zu prüfenden Substanzen in den entsprechenden Konzentrationen eingemischt. Die behandelte und infestierte Erde wird dann

in 1c Töpfe (180 ccm) gefüllt und jeweils drei Sojabohen (cv. Maple Arrow) eingesät. Jede Behandlung wird dreimal wiederholt. Die Töpfe werden bei ca 27°C für vier bis fünf Wochen im Gewächshaus inkubiert. Die Pflanzen werden dann vorsichtig den Töpfen entnommen, die Wurzeln gewaschen und die Anzahl der Zysten bestimmt

Die Verbindungen 1 und 2 aus der Tabelle zeigen gegen Heterodera glycines eine gute Wirkung, was sich in der fast vollständigen Reduktion der Zystenbildung zeigt.

## Patentansprüche (Patentansprüche für folgende(n) Vertragsstaat(en): AT, BE, CH, DE, DK, FR, GB, GR, IT, LI, LU, NL, SE)

1. Verfahren zur Bekämpfung oder Verhütung des Befalls von Pflanzen durch Nematoden, dadurch gekennzeichnet, dass man als aktive Komponente eine Verbindung der Formel I 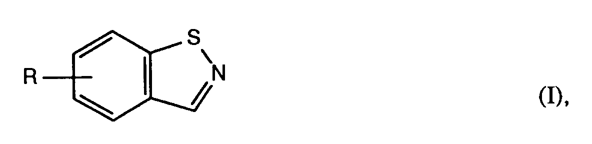 worin R Nitro oder Halogen bedeutet, auf die Pflanze oder deren Standort appliziert.

2. Verfahren gemäss Anspruch 1, dadurch gekennzeichnet, dass als aktive Komponente eine Verbindung der Formel I' appliziert wird, 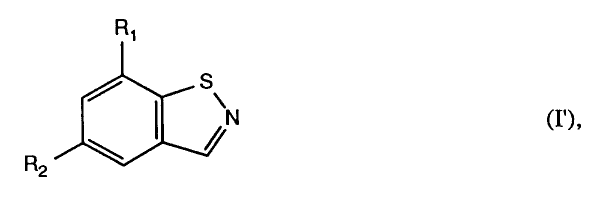 worin
R₁ Wasserstoff oder Halogen bedeutet,
R₂, wenn R₁ für Wasserstoff steht, Nitro, wenn R₁ für Halogen steht, Wasserstoff bedeutet.

3. Verfahren gemäss Anspruch 2, dadurch gekennzeichnet, dass als aktive Komponente eine Verbindung der Formel Ia appliziert wird, 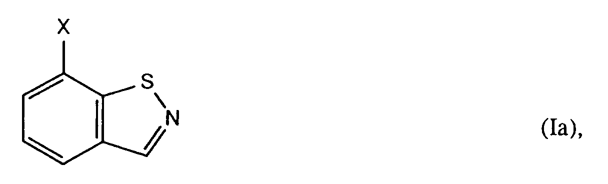 worin
X Brom, Fluor oder Jod bedeutet.

4. Verfahren gemäss Anspruch 1, dadurch gekennzeichnet, dass als aktive Komponente 5-Nitrobenzisothiazol appliziert wird.

5. Verfahren gemäss Anspruch 1, dadurch gekennzeichnet, dass als aktive Komponente 7-Chlorbenzisothiazol appliziert wird.

6. Halo-benzisothiazole der Formel I'' 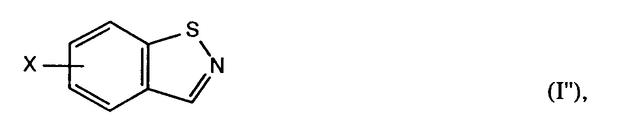 dadurch gekennzeichnet, dass X Brom, Fluor oder Jod bedeutet.

7. 7-Halo-benzisothiazole der Formel Ia 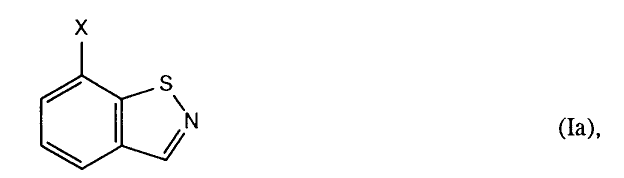 gemäss Anspruch 6.

8. 6-Halo-benzisothiazole der Formel I(c) 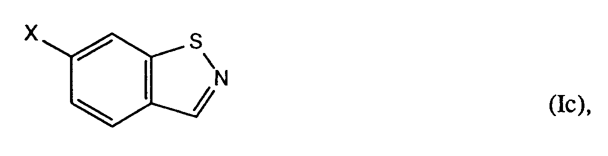 gemäss Anspruch 6.

9. 5-Halo-benzisothiazole der Formel I(d) 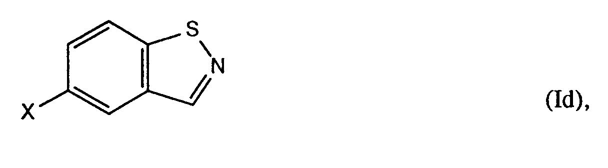 gemäss Anspruch 6.

10. 4-Halo-benzisothiazole der Formel I(e) 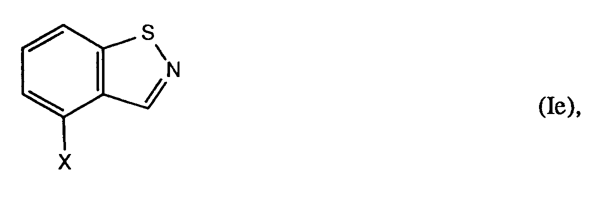 gemäss Anspruch 6.

11. Verfahren zur Herstellung der Verbindungen der Formel I'' gemäss Anspruch 6, 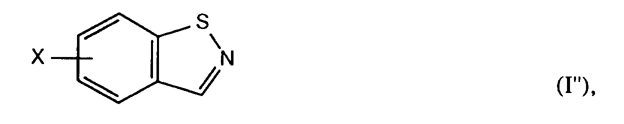 worin X Brom, Fluor oder Jod bedeutet, dadurch gekennzeichnet, dass man das ein Aminobenzisothiazol der Formel II zum Diazoniumsalz der Formel III diazotiert und letzeres in Gegenwart von Cu(I)X oder Fe(II)X₂, worin X Brom, Fluor oder Jod bedeutet, mit der entsprechenden Halogenwasserstoffsäure der Formel HX umsetzt,

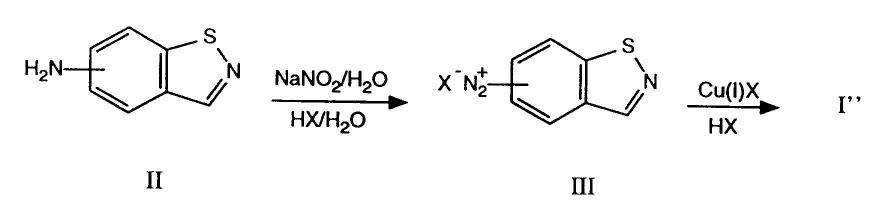

12. Verfahren gemäss Anspruch 11 zur Herstellung der Verbindungen der Formel Ia,

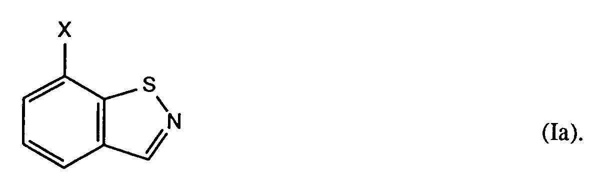

13. Verfahren zur Herstellung von Verbindungen der Formel I'' 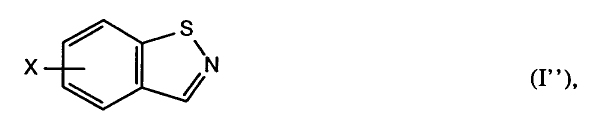 worin
X Brom, Fluor oder Jod bedeutet, dadurch gekennzeichnet, dass man ein substituiertes Halo-benzaldehyd der Formel V mit einem Thioalkohol R₉SH in Lösung, in Gegenwart einer Base zum Thioether der Formel VI umsetzt und letzteren, mit oder ohne Isolierung, mit Hydroxylamin-O-sulfonsäure in Lösung umsetzt und zum erwünschten Produkt zyklisiert, 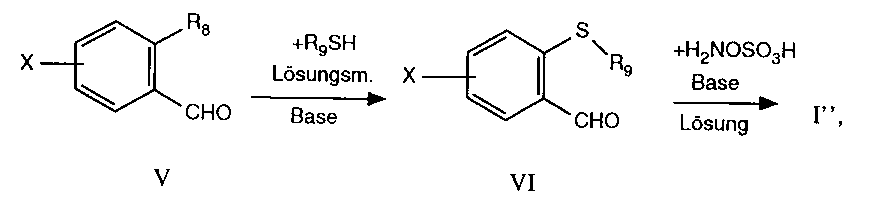 wobei X Brom, Fluor oder Jod, R₈ Halogen und R₉ C₁-C₃₀-Alkyl, C₃-C₇-Cycloalkyl oder C₇-C₉-Aralkyl bedeuten.

14. Verfahren gemäss Anspruch 13, dadurch gekennzeichnet, dass man den Thioether der Formel VI mit Hydroxylamin-O-sulfonsäure in Lösung umsetzt und durch Zugabe einer Base zum erwünschten Produkt zyklisiert.

15. Verfahren zur Herstellung von Verbindungen der Formel Ia 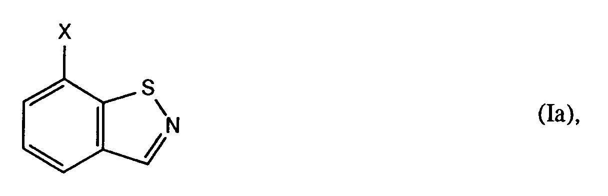 gemäss Anspruch 13,
dadurch gekennzeichnet, dass man ein 2,3-Dihalo-benzaldehyd der Formel VII mit Benzylmercaptan in Lösung, in Gegenwart einer Base zum Thioether der Formel VIII umsetzt und letzteren, mit oder ohne Isolierung, mit Hydroxylamin-O-sulfonsäure in Lösung umsetzt und zum erwünschten Produkt zyklisiert, 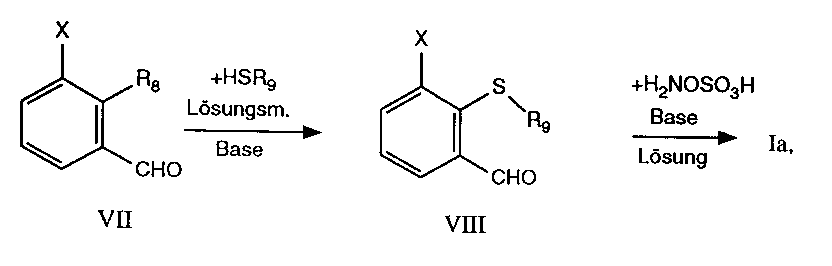 worin X Brom, Fluor oder Jod, R₈ Halogen und R₉, den Benzylrest bedeuten.

16. Verfahren gemäss Anspruch 15, dadurch gekennzeichnet, dass man den Thioether der Formel VIII durch Zugabe einer Base zum erwünschten Produkt zyklisiert.

17. Verfahren gemäss Anspruch 16, dadurch gekennzeichnet, dass das Zwischenprodukt der Formel VIII mit Hydroxylamin-O-sulfonsäure bei pH = 5 umgesetzt wird und die Zyklisierung durch anschliessende Base-Zugabe durchgeführt wird.

18. Verbindungen der Formel VI 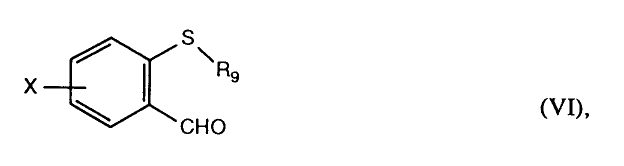 worin
X Brom, Fluor oder Jod, R Halogen oder Nitro und R₉ C₁-C₃₀-Alkyl, C₃-C₇-Cycloalkyl oder C₇-C₉-Aralkyl bedeuten.

19. Verbindungen gemäss Anspruch 18, worin R₉ C₁-C₆-Alkyl oder den Benzylrest bedeutet.

20. Verbindungen der Formel VIII 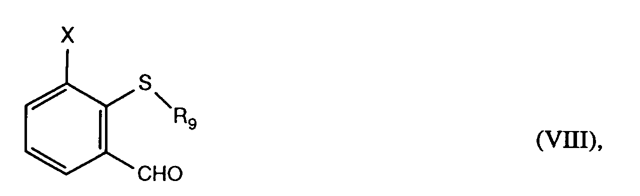 gemäss Anspruch 19, worin
X Brom, Fluor oder Jod bedeutet.

21. Verfahren gemäss Anspruch 1, dadurch gekennzeichnet, dass es sich bei den Nematoden um pflanzenparasitäre Arten handelt.

22. Verfahren gemäss Anspruch 21 gegen Nematoden der Gattung Heterodera.

23. Verfahren gemäss Anspruch 21 gegen Nematoden der Gattung Meloidogyne.

## Patentansprüche (Patentansprüche für folgende(n) Vertragsstaat(en): ES)

1. Verfahren zur Bekämpfung oder Verhütung eines Befalls von Kulturpflanzen durch Nematoden, dadurch gekennzeichnet, dass man eine Verbindung der Formel I 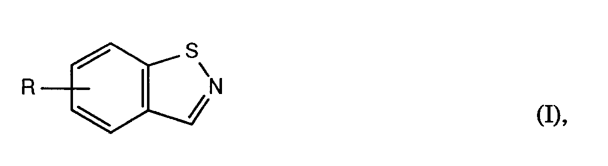 worin R Nitro oder Halogen bedeutet, auf die Pflanze oder deren Standort appliziert.

2. Verfahren gemäss Anspruch 1, dadurch gekennzeichnet, dass es sich bei den Nematoden um pflanzenparasitäre Arten handelt.

3. Verfahren gemäss Anspruch 2 gegen Nematoden der Gattung Heterodera.

4. Verfahren gemäss Anspruch 3 gegen Nematoden der Gattung Meloidogyne.

5. Verfahren gemäss Anspruch 1, worin eine Verbindung der Formel I' appliziert wird, 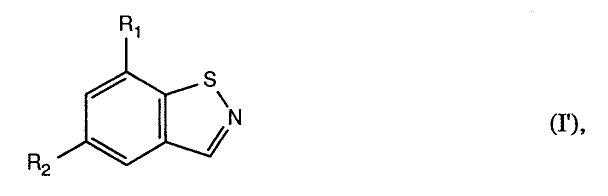 worin
R₁ Wasserstoff oder Halogen bedeutet,
R₂, wenn R₁ für Wasserstoff steht, Nitro, wenn R₁ für Halogen steht, Wasserstoff bedeutet.

6. Verfahren gemäss Anspruch 5, worin eine Verbindung der Formel Ia appliziert wird, 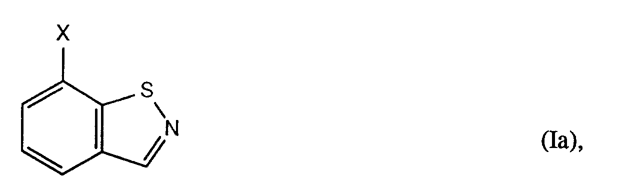 worin
X Brom, Fluor oder Jod bedeutet.

7. Verfahren gemäss Anspruch 1, worin 5-Nitrobenzisothiazol appliziert wird.

8. Verfahren gemäss Anspruch 1, worin 7-Chlorbenzisothiazol appliziert wird.

9. Verfahren zur Herstellung der Verbindungen der Formel I'' 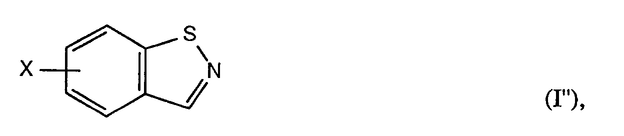 worin X Brom, Fluor oder Jod bedeutet, dadurch gekennzeichnet, dass man das ein Aminobenzisothiazol der Formel II zum Diazoniumsalz der Formel III diazotiert und letzeres in Gegenwart von Cu(I)X oder Fe(II)X₂, worin X Brom, Fluor oder Jod bedeutet, mit der entsprechenden Halogenwasserstoffsäure der Formel HX umsetzt,

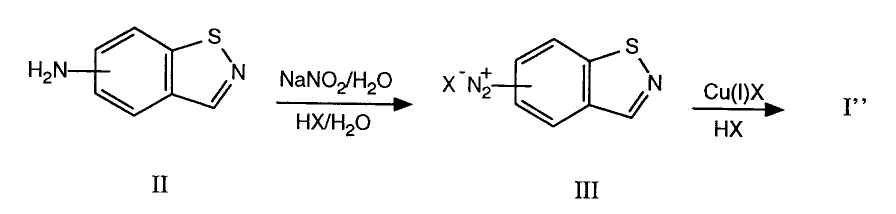

10. Verfahren gemäss Anspruch 9 zur Herstellung der Verbindungen der Formel Ia,

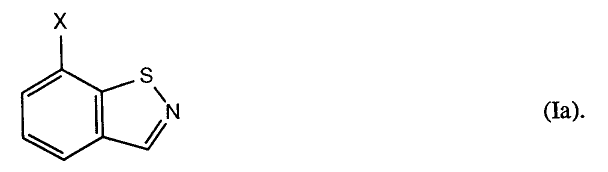

11. Verfahren zur Herstellung von Verbindungen der Formel I'' 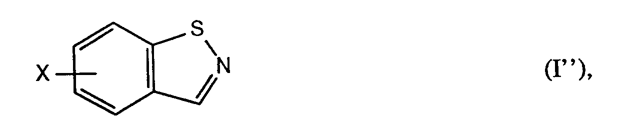 worin
X Brom, Fluor oder Jod bedeutet, dadurch gekennzeichnet, dass man ein substituiertes Halo-benzaldehyd der Formel V mit einem Thioalkohol R₉SH in Lösung, in Gegenwart einer Base zum Thioether der Formel VI umsetzt und letzteren, mit oder ohne Isolierung, mit Hydroxylamin-O-Sulfonsäure in Lösung umsetzt und zum erwünschten Produkt zyklisiert, 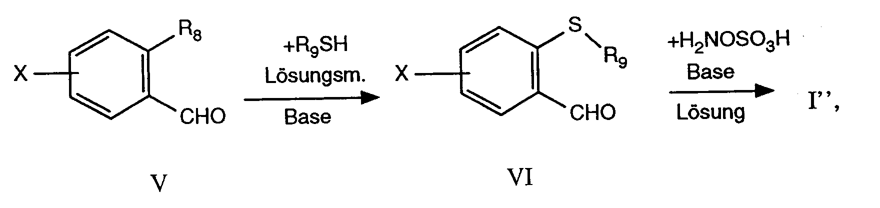 wobei X Brom, Fluor oder Jod, R₈ Halogen und R₉ C₁-C₃₀-Alkyl, C₃-C₇-Cycloalkyl oder C₇-C₉-Aralkyl bedeuten.

12. Verfahren gemäss Anspruch 11, dadurch gekennzeichnet, dass man den Thioether der Formel VI mit Hydroxylamin-O-sulfonsäure in Lösung umsetzt und durch Zugabe einer Base zum erwünschten Produkt zyklisiert.

13. Verfahren zur Herstellung von Verbindungen der Formel Ia gemäss Anspruch 11 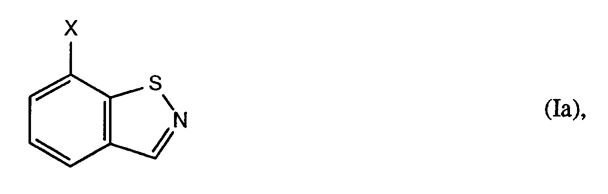 dadurch gekennzeichnet, dass man ein 2,3-Dihalo-benzaldehyd der Formel VII mit Benzylmercaptan in Lösung, in Gegenwart einer Base zum Thioether der Formel VIII umsetzt und letzteren, mit oder ohne Isolierung, mit Hydroxylamin-O-sulfonsäure in Lösung umsetzt und zum erwünschten Produkt zyklisiert, 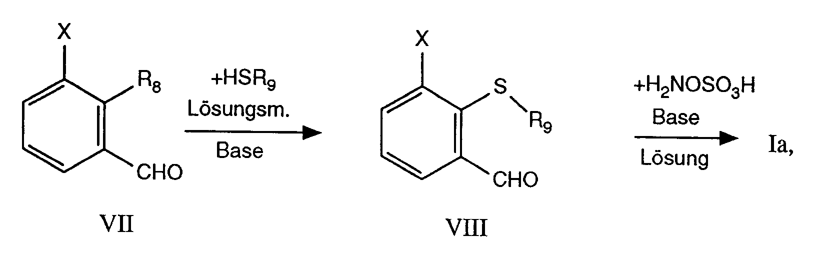 worin X Brom, Fluor oder Jod, R₈ Halogen und R₉ den Benzylrest bedeuten.

14. Verfahren gemäss Anspruch 13, dadurch gekennzeichnet, dass man den Thioether der Formel VIII durch Zugabe einer Base zum erwünschten Produkt zyklisiert.

15. Verfahren gemäss Anspruch 14, dadurch gekennzeichnet, dass das Zwischenprodukt der Formel VIII mit Hydroxylamin-O-sulfonsäure bei pH = 5 umgesetzt wird und die Zyklisierung durch anschliessende Base-Zugabe durchgeführt wird.

## Claims (Claims for the following Contracting State(s): AT, BE, CH, DE, DK, FR, GB, GR, IT, LI, LU, NL, SE)

1. A method of controlling or preventing an attack on plants by nematodes, which comprises applying to the plant or to the locus thereof as active ingredient a compound of formula I 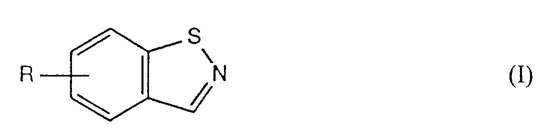 wherein R is nitro or halogen.

2. A method according to claim 1 wherein there is applied as active ingredient a compound of formula I' 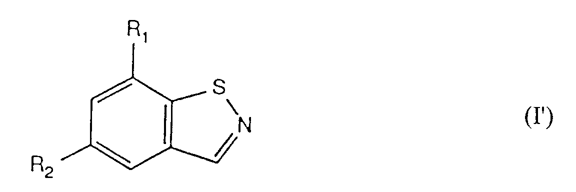 wherein R₁ is hydrogen or halogen, and R₂ is nitro when R₁ is hydrogen and is hydrogen when R₁ is halogen.

3. A method according to claim 2 wherein there is applied as active ingredient a compound of formula Ia 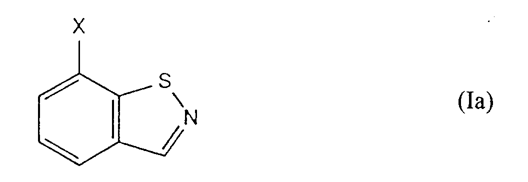 wherein X is bromine, fluorine or iodine.

4. A method according to claim 1 wherein 5-nitrobenzisothiazole is applied as active ingredient.

5. A method according to claim 1 wherein 7-chlorobenzisothiazole is applied as active ingredient.

6. A halo-benzisothiazole of formula I'' 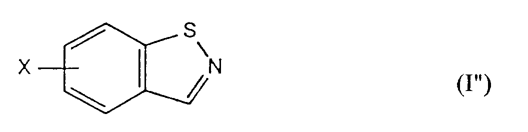 wherein X is bromine, fluorine or iodine.

7. A 7-halo-benzisothiazole of formula Ia 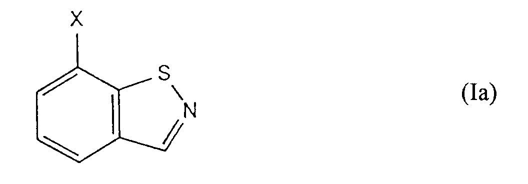 according to claim 6.

8. A 6-halo-benzisothiazole of formula I(c) 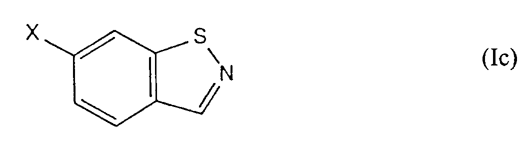 according to claim 6.

9. A 5-halo-benzisothiazole of formula I(d) 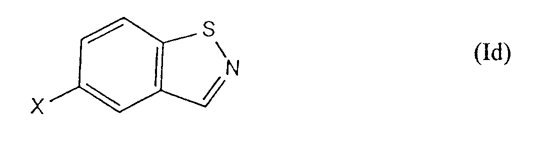 according to claim 6.

10. A 4-halo-benzisothiazole of formula I(e) 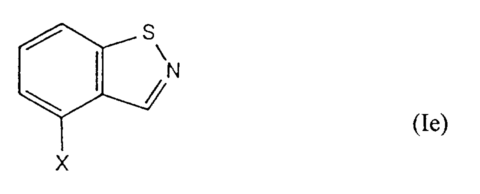 according to claim 6.

11. A process for the preparation of a compound of formula I'' according to claim 6 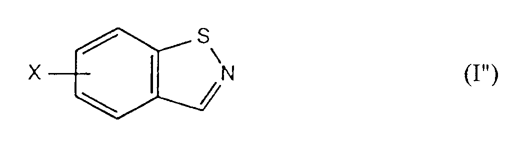 wherein X is bromine, fluorine or iodine, which process comprises diazotising an amino-benzisothiazole of formula II to form the diazonium salt of formula III and reacting the latter in the presence of Cu(I)X or Fe(II)X₂, wherein X is bromine, fluorine or iodine, with the corresponding hydrohalic acid of the formula HX,

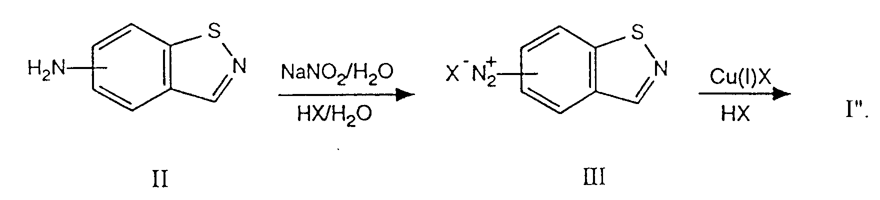

12. A process according to claim 11 for the preparation of a compound of formula Ia

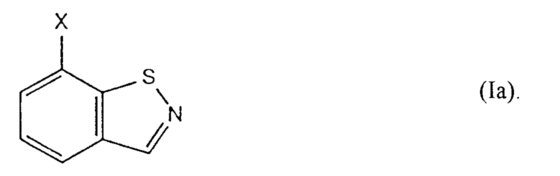

13. A process for the preparation of a compound of formula I'' 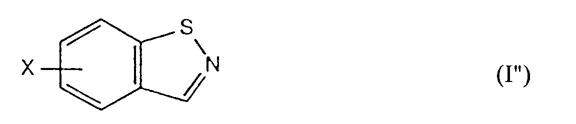 wherein X is bromine, fluorine or iodine, which process comprises reacting a substituted halobenzaldehyde of formula V with a thioalcohol R₉SH in solution, in the presence of a base, to form the thioether of formula VI, which is then reacted, with or without isolation, with hydroxylamine-O-sulfonic acid in solution and cyclised to form the desired product, 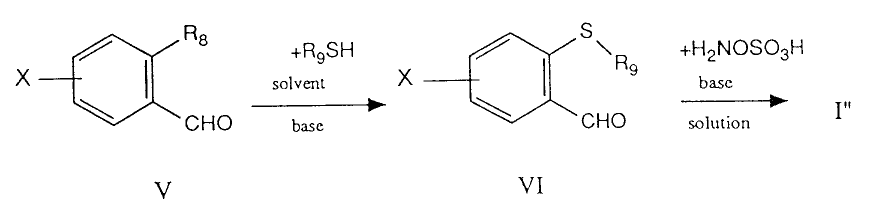 wherein X is bromine, fluorine or iodine, R₈ is halogen and R₉ is C₁-C₃₀alkyl, C₃-C₇cycloalkyl or C₇-C₉aralkyl.

14. A process according to claim 13 wherein the thioether of formula VI is reacted with hydroxylamine-O-sulfonic acid in solution, and cyclised to form the desired product by the addition of a base.

15. A process for the preparation of a compound of formula Ia 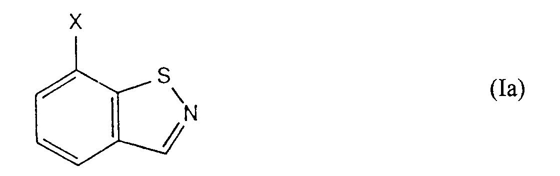 according to claim 13, which process comprises reacting a 2,3-dihalobenzaldehyde of formula VII with benzylmercaptan in solution, in the presence of a base, to form the thioether of formula VIII, which is then reacted, with or without isolation, with hydroxylamine-O-sulfonic acid in solution and cyclised to form the desired product, 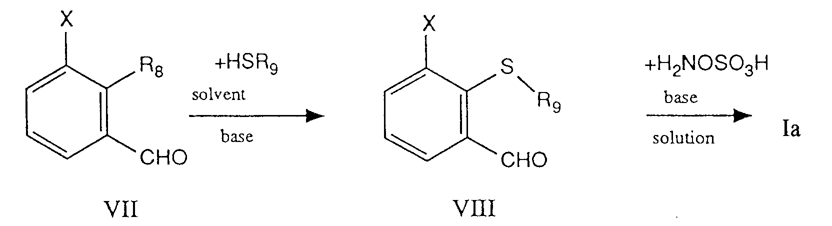 wherein X is bromine, fluorine or iodine, R₈ is halogen and R₉ is a benzyl radical.

16. A process according to claim 15 wherein the thioether of formula VIII is cyclised to the desired product by the addition of a base.

17. A process according to claim 16 wherein the intermediate of formula VIII is reacted with hydroxylamine-O-sulfonic acid at pH = 5 and the cyclisation is effected by the subsequent addition of a base.

18. A compound of formula VI 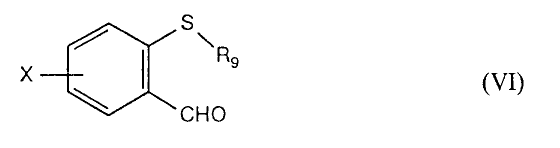 wherein X is bromine, fluorine or iodine, R is halogen or nitro and R₉ is C₁-C₃₀alkyl, C₃-C₇-cycloalkyl or C₇-C₉aralkyl.

19. A compound according to claim 18 wherein R₉ is C₁-C₆alkyl or a benzyl radical.

20. A compound of formula VIII 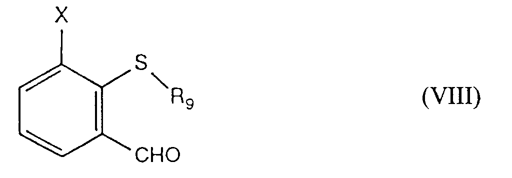 according to claim 19 wherein X is bromine, fluorine or iodine.

21. A method according to claim 1 wherein the nematodes are species that are parasites of plants.

22. A method according to claim 1 against nematodes of the genus Heterodera.

23. A method according to claim 1 against nematodes of the genus Meloidogyne.

## Claims (Claims for the following Contracting State(s): ES)

1. A method of controlling or preventing an attack on cultivated plants by nematodes, which comprises applying to the plant or to the locus thereof a compound of formula I 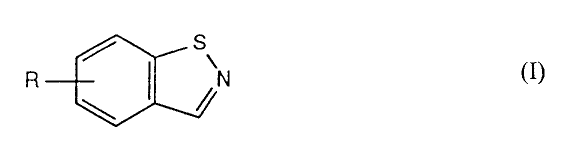 wherein R is nitro or halogen.

2. A method according to claim 1 wherein the nematodes are species that are parasites of plants.

3. A method according to claim 2 against nematodes of the genus Heterodera.

4. A method according to claim 3 against nematodes of the genus Meloidogyne.

5. A method according to claim 1 wherein there is applied a compound of formula I' 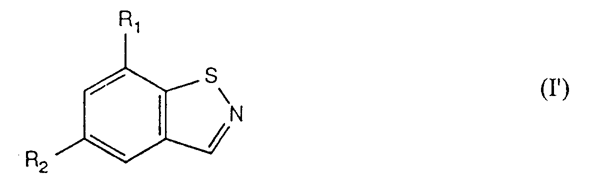 wherein R₁ is hydrogen or halogen, and R₂ is nitro when R₁ is hydrogen and is hydrogen when R₁ is halogen.

6. A method according to claim 5 wherein there is applied a compound of formula Ia 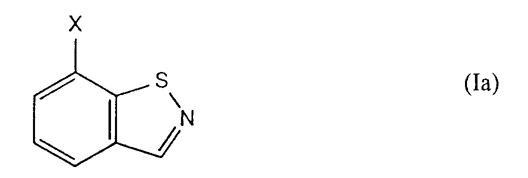 wherein X is bromine, fluorine or iodine.

7. A method according to claim 1 wherein 5-nitrobenzisothiazole is applied.

8. A method according to claim 1 wherein 7-chlorobenzisothiazole is applied.

9. A process for the preparation of a compound of formula I'' 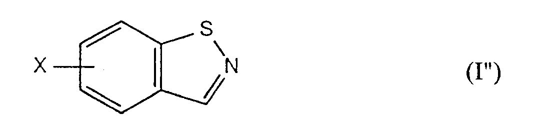 wherein X is bromine, fluorine or iodine, which process comprises diazotising an aminobenzisothiazole of formula II to form the diazonium salt of formula III and reacting the latter in the presence of Cu(I)X or Fe(II)X₂, wherein X is bromine, fluorine or iodine, with the corresponding hydrohalic acid of the formula HX,

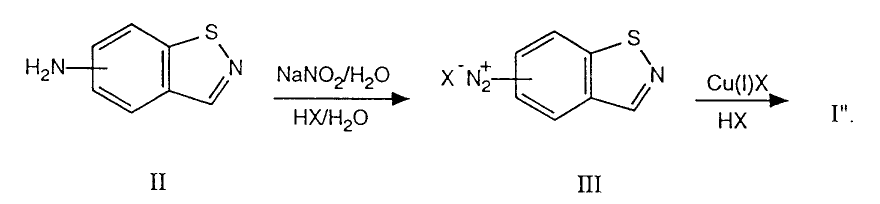

10. A process according to claim 9 for the preparation of a compound of formula Ia

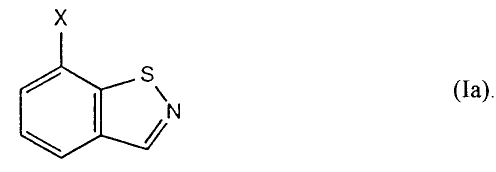

11. A process for the preparation of a compound of formula I'' 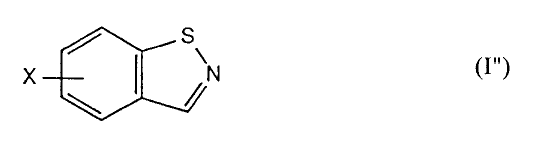 wherein X is bromine, fluorine or iodine, which process comprises reacting a substituted halobenzaldehyde of formula V with a thioalcohol R₉SH in solution, in the presence of a base, to form the thioether of formula VI, which is then reacted, with or without isolation, with hydroxylamine-O-sulfonic acid in solution and cyclised to form the desired product, 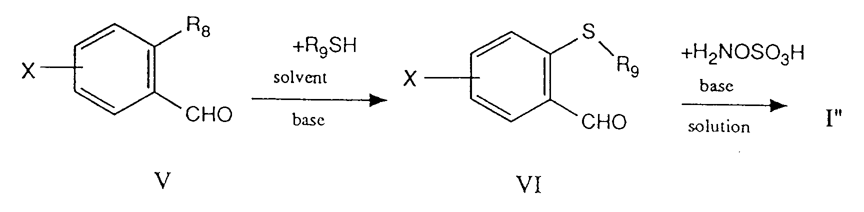 wherein X is bromine, fluorine or iodine, R₈ is halogen and R₉ is C₁-C₃₀alkyl, C₃-C₇cycloalkyl or C₇-C₉aralkyl.

12. A process according to claim 11 wherein the thioether of formula VI is reacted with hydroxylamine-O-sulfonic acid in solution, and cyclised to form the desired product by the addition of a base.

13. A process for the preparation of a compound of formula Ia according to claim 11 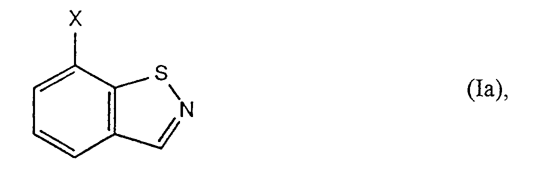 which process comprises reacting a 2,3-dihalobenzaldehyde of formula VII with benzylmercaptan in solution, in the presence of a base, to form the thioether of formula VIII, which is then reacted, with or without isolation, with hydroxylamine-O-sulfonic acid in solution and cyclised to form the desired product, 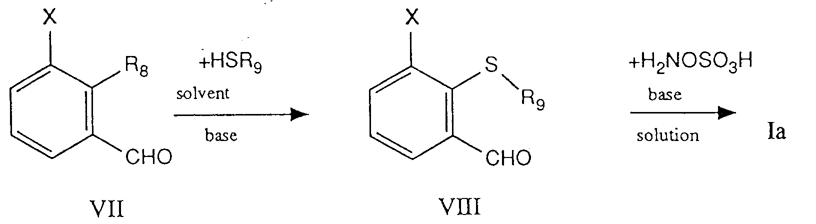 wherein X is bromine, fluorine or iodine, R₈ is halogen and R₉ is a benzyl radical.

14. A process according to claim 13 wherein the thioether of formula VIII is cyclised to the desired product by the addition of a base.

15. A process according to claim 14 wherein the intermediate of formula VIII is reacted with hydroxylamine-O-sulfonic acid at pH = 5 and the cyclisation is effected by the subsequent addition of a base.

## Revendications (Revendications pour l'(les) Etat(s) contractant(s) suivant(s): AT, BE, CH, DE, DK, FR, GB, GR, IT, LI, LU, NL, SE)

1. Procédé de lune ou de prévention de l'attaque des plantes par les nématodes, caractérisé en ce qu'on applique comme composé actif un composé de formule I 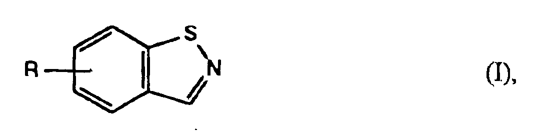 dans laquelle R représente un nitro ou un halogène, sur les plantes ou à l'endroit où elles se trouvent.

2. Procédé selon la revendication 1, caractérisé en ce qu'on applique comme composant actif un composé dc formule I' 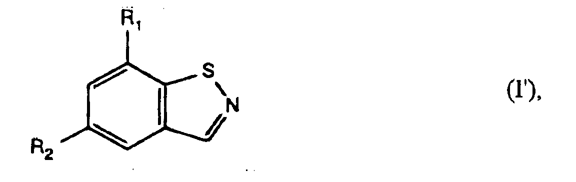 dans laquelle
R₁ représente un hydrogène ou un halogène,
R₂, lorsque R₁ représente un hydrogène, représente un nitro, et, lorsque R₁ représente un halogène, R₂ représente un hydrogène.

3. Procédé selon la revendication 2, caractérisé en ce qu'on applique comme composant actif un composé de formule la 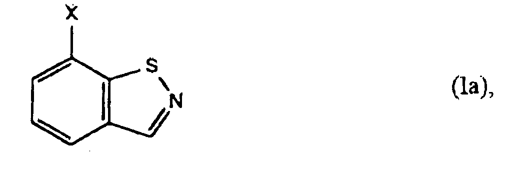 dans laquelle,
X représente le brome, le fluor ou l'iode.

4. Procédé selon la revendication 1, caractérisé en ce qu'on applique comme composant actif le 5-nitrobenzisothiazole.

5. Procédé selon la revendication 1, caractérisé ai ce qu'on applique comme composant actif le 7-chlorobenzisothiazole.

6. Halo-benzisothiazoles de formule I'' 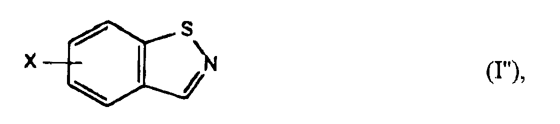 caractérisée en ce que X représente le brome, le fluor ou l'iode.

7. 7-halo-benzisothiazoles de formule Ia 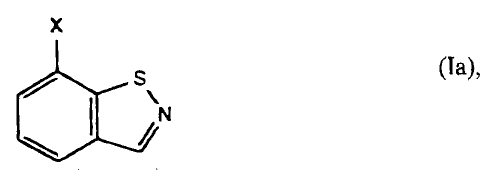 selon la revendication 6.

8. 6-halo-benzisothiazoles de formule Ic 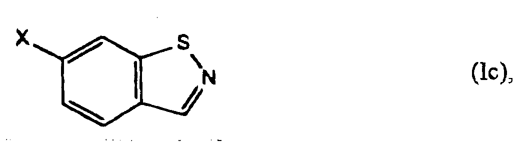 selon la revendication 6.

9. 5-halo-benzisothiazoles de formule Id 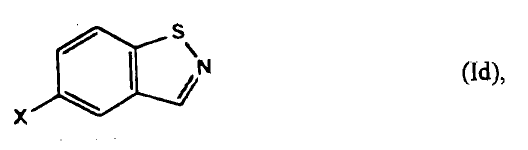 selon la revendication 6.

10. 4-halo-benzisothiazoles de formule Ie 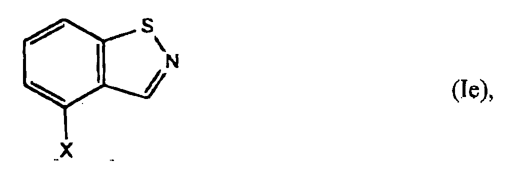 selon la revendication 6.

11. Procédé de préparation des composés de formule I'' selon la revendication 6, 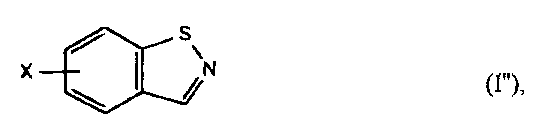 dans laquelle X représente le brome, le fluor ou l'iode, caractérisé en ce qu'on diazote un aminobenzisothiazole de formule II en un sel de diazonium de formule III et en ce qu'on fait réagir ce dernier en présence de Cu(I)X ou Fe(II)X₂, où X représente le brome, le fluor ou l'iode, avec l'acide halohydrique correspondant dc formule HX,

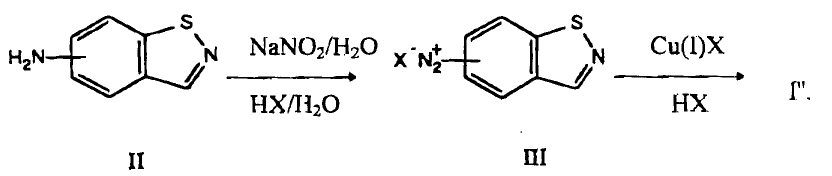

12. Procédé selon la revendication 11 de préparation des composes de formule Ia,

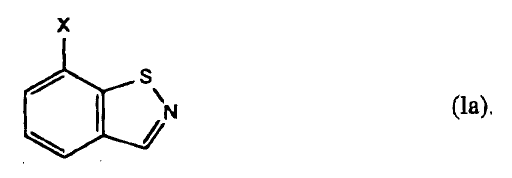

13. Procédé de préparation de composés de formule I'' 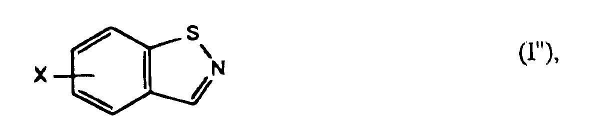 dans laquelle X représente le brome, le fluor ou l'iode, caractérise en ce qu'on fait réagir un halo-benzaldéhyde substitué de formule V avec un thioalcool R₉SH en solution, en présence d'une base, pour donner le thioéther de formule VI, et en ce qu'on fait réagir ce dernier avec ou sans isolement, avec de l'acide hydroxylamine-O-Sulfonique en solution et en ce qu'un le cyclise pour obtenir le produit désiré, 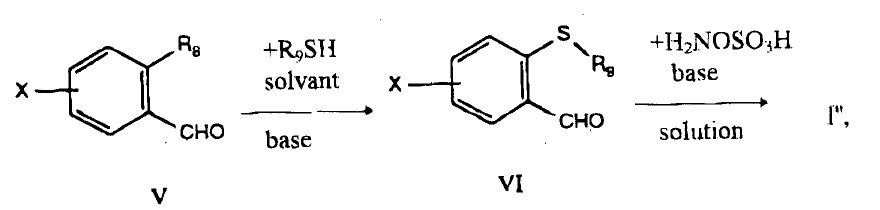 où X représente le brome, le fluor ou l'iode, R₈ représente un halogène et R₉ représente un alkyle en C₁ à C₃₀, un cycloalkyle en C₃ à C₇ ou un aralkyle en C₇ à C₉.

14. Procédé selon la revendication 3, caractérisé en ce qu'on fait réagir le thioéther de formule VI avec l'acide hydroxylamine-O-sulfonique et cri ce qu'on le cyclise, par addition d'une base, pour obtenir le produit désiré.

15. Procédé de préparation de composés de formule la 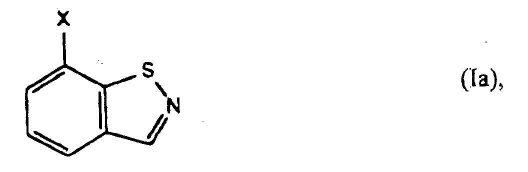 selon la revendication 13,
caractérisé en ce qu'on fait réagir un 2,3-dihalo-benzaldéhyde de formule VII avec du benzylmercaptan en solution, en présence d'une base, pour donner le thioéther de formule VIII, et en ce qu'on fait réagir ce dernier, avec ou sans isolement, avec de l'acide hydroxylamine-O-sulfonique en solution et en ce qu'on le cyclise pour donner le produit désiré, 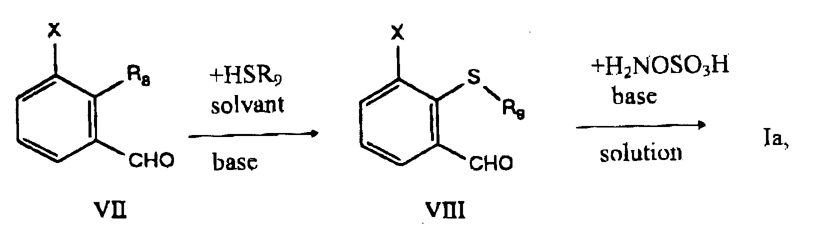 où X représente le brome, le fluor ou l'iode, R₈ représente un halogène et R₉ le radical benzyle.

16. Procédé selon la revendication 15, caractérisé en ce qu'on cyclise le thioéther de formule VIII par addition d'une base pour donner le produit désiré.

17. Procédé selon la revendication 16, caractérisé en ce qu'on fait réagir le produit intermédiaire de formule VIII avec l'acide hydroxylamine-O-sulfonique à pH 5 et en ce qu'on conduit la cyclisation par addition ultérieure d'une base.

18. Composés de formule VI 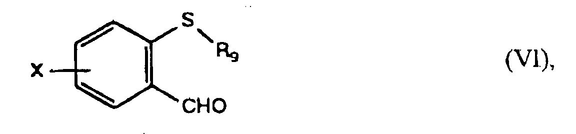 dans laquelle
X représente le brome, le fluor ou l'iode, R représente un halogène ou un nitro et R₉ représente un alkyle en C₁ à C₃₀, un cycloalkyle en C₃ à C₇ ou un aralkyle en C₇ à C₉.

19. Composés selon la revendication 18, où R₉ représente un alkyle en C₁ à C₆ ou le radical benzyle.

20. Composés dc formule VIII 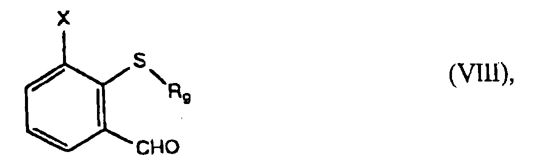 selon la revendication 19, dans laquelle
X représente le brome, le fluor ou l'iode.

21. Procédé selon la revendication 1, caractérisé en ce que, pour ce qui est des nématodes, il s'agit des espèces phytoparasitaires.

22. Procédé selon la revendication 21, contre les nématodes du genre Heterodera.

23. Procédé selon la revendication 21, contre les nématodes du genre Meloïdogyne.

## Revendications (Revendications pour l'(les) Etat(s) contractant(s) suivant(s): ES)

1. Procédé pour combattre ou prévenir de l'attaque des plantes par les nématodes, caractérisé en ce qu'on applique un composé de formule I 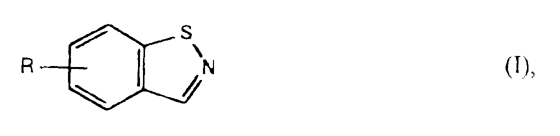 dans laquelle R représente un nitro ou un halogène, sur les plantes ou à l'endroit où elles se trouvent.

2. Procédé selon la revendication 1, caractérisé en ce que, pour ce qui est des nématodes, il s'agit des espèces phytoparasitaires,

3. Procédé selon la revendication 2 contre les nématodes du genre Heterodera.

4. Procédé selon la revendication 3 contre les nématodes du genre Meloïdogyne.

5. Procédé selon la revendication 1, caractérisé en ce qu'on applique un composé de formule I' 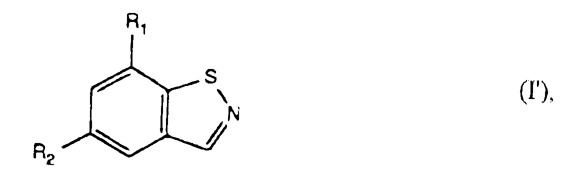 dans laquelle
R₁ représente un hydrogène ou un halogène,
R₂, lorsque R₁ représente un hydrogène, représente un nitro, et, lorsque R₁ représente un halogène, R₂ représente un hydrogène.

6. Procédé selon la revendication 5, caractérisé en ce qu'ou applique un composé de formule Ia 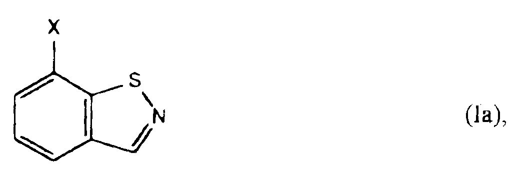 dans laquelle,
X représente le brome, le fluor ou l'iode.

7. Procédé selon la revendication 1, dans lequel on applique le 5-nitrobenzisothiazole.

8. Procédé selon la revendication 1, dans lequel on applique le 7-chlorobenzisothiazole.

9. Procédé de préparation des composés de formule I'' 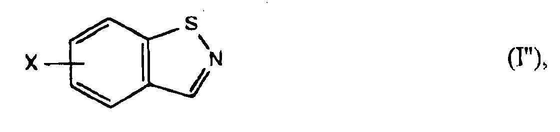 dans laquelle X représente le brome, le fluor ou l'iode, caractérisé en ce qu'on diazote un aminobenzisothiazole de formule II pour donner le sel de diazonium de formule III et en ce qu'on fait réagir ce dernier en présence de Cu(I)X ou dc Fe(II)X₂ , où X représente le brome, le fluor ou l'iode, avec l'acide halogénhydrique de formule HX correspondant,

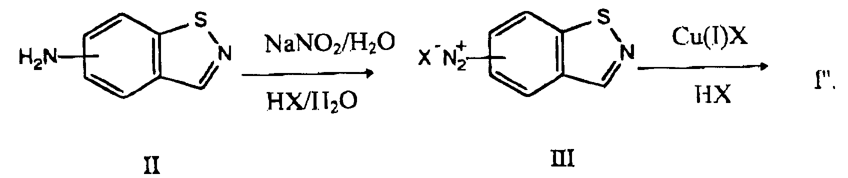

10. Procédé selon la revendication 9 de préparation des composés de formule la,

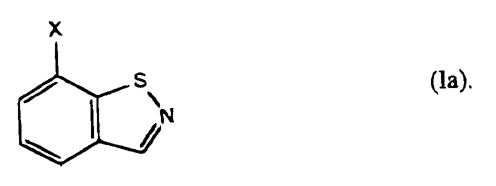

11. Procédé de préparation de composés de formule I'' 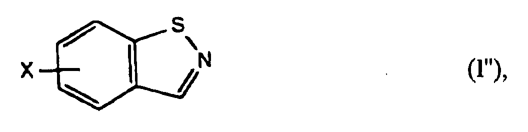 dans laquelle X représente le brome, le fluor ou l'iode, caractérisé en ce qu'on fait réagir un halo-benzaldéhyde substitué de formule V avec un thioalcool R₉SH en solution, en présence d'une base, pour donner le thioéther de formule VI, et en ce qu'on fait réagir ce dernier avec ou sans isolement, avec de l'acide hydroxylamine-O-sulfonique en solution et en ce qu'on le cyclise pour obtenir le produit désiré, 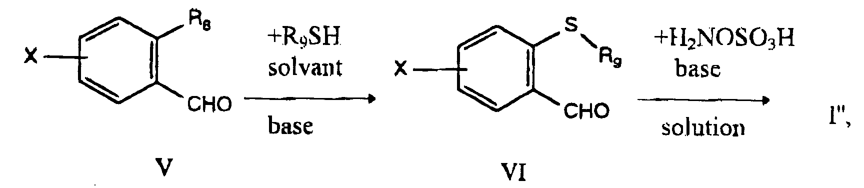 où X représente le brome, le fluor ou l'iode, R₈ représente un halogène et R₉ représente un alkyle en C₁ à C₃₀, un cycloalkyle en C₃ à C₇ ou un aralkyle en C₇ à C₉.

12. Procédé selon la revendication 11, caractérisé en ce qu'on fait réagir le thioéther de formule VI avec de l'acide hydroxylamine-O-sulfonique et en ce qu'on le cyclise, par addition d'une base, pour obtenir le produit désiré.

13. Procédé de préparation de composés de formule la selon la revendication 11 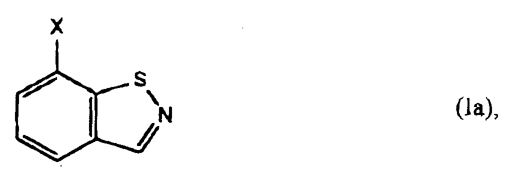 caractérisé on ce qu'on fait réagir un 2,3-dihalo-benzaldéhyde de formule VII avec du benzylmercaptan en solution, en présence d'une base, pour donner le thioéther de formule VIII, et on ce qu'on fait réagir ce dernier, avec ou sans isolement, avec de l'acide hydroxylamine-O-sulfonique en solution et on ce qu'on le cyclise pour donner le produit désiré, 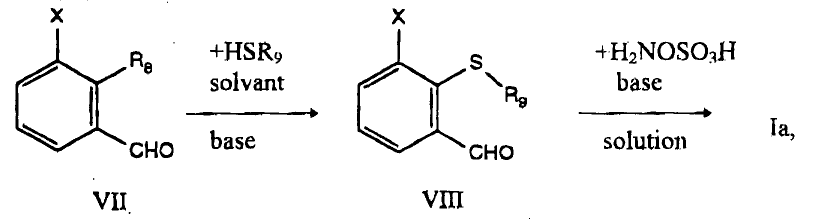 où X représente le brome, le fluor ou l'iode, R₈ représente un halogène et R₉ le radical benzyle.

14. Procédé selon la revendication 13, caractérisé en ce qu'on cyclise le thioéther de formule VIII par addition d'une base pour donner le produit désiré.

15. Procédé selon la revendication 14, caractérisé en ce qu'on fait réagir le produit intermédiaire de formule VIII avec de l'acide hydroxylamine-O-sulfonique à pH 5 et en ce qu'on conduit la cyclisation par addition ultérieure d'une base.
